(19) Europäisches Patentamt / European Patent Office / Office européen des brevets

(11) **EP 2 975 389 B1**

(12) **EUROPÄISCHE PATENTSCHRIFT**

(45) Veröffentlichungstag und Bekanntmachung des Hinweises auf die Patenterteilung:
**28.07.2021 Patentblatt 2021/30**

(51) Int Cl.:
*G01N 27/22* (2006.01)    *G01N 33/44* (2006.01)

(21) Anmeldenummer: **15169293.6**

(22) Anmeldetag: **27.05.2015**

(54) **VERFAHREN UND VORRICHTUNG ZUR ZERSTÖRUNGSFREIEN UND BERÜHRUNGSLOSEN ERMITTLUNG DES ALTERUNGS- UND/ODER ERMÜDUNGSZUSTANDS EINES EIN ELASTOMER ENTHALTENDEN BAUTEILS**

METHOD AND DEVICE FOR NON- DESTRUCTIVE AND CONTACTLESS DETERMINATION OF THE AGING AND/OR FATIGUE OF A COMPONENT CONTAINING ELASTOMER

PROCEDE ET DISPOSITIF DE DETERMINATION NON DESTRUCTIF ET SANS CONTACT DE L'ETAT DE VIEILLISSEMENT ET/OU DE FATIGUE D'UN ELEMENT CONTENANT DE L'ELASTOMERE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.07.2014 DE 102014213969**

(43) Veröffentlichungstag der Anmeldung:
**20.01.2016 Patentblatt 2016/03**

(73) Patentinhaber: **ContiTech Luftfedersysteme GmbH**
**30165 Hannover (DE)**

(72) Erfinder:
• **Wietzke, Steffen**
**31275 Lehrte (DE)**
• **Reck, Siegfried**
**31582 Nienburg (DE)**
• **Sostmann, Stefan**
**30855 Langenhagen (DE)**

(74) Vertreter: **Continental Corporation**
**c/o Continental AG**
**Intellectual Property**
**Postfach 169**
**30001 Hannover (DE)**

(56) Entgegenhaltungen:
EP-A2- 2 375 099    DE-A1-102010 016 359
DE-U1-202013 000 810    US-A- 3 906 340
US-A1- 2004 159 383

• **MINWOO PARK ET AL: "Highly stretchable electric circuits from a composite material of silver nanoparticles and elastomeric fibres", NATURE NANOTECHNOLOGY, Bd. 7, Nr. 12, 25. November 2012 (2012-11-25), Seiten 803-809, XP055227222, GB ISSN: 1748-3387, DOI: 10.1038/nnano.2012.206**
• **J. P. LYNCH: "A Summary Review of Wireless Sensors and Sensor Networks for Structural Health Monitoring", THE SHOCK AND VIBRATION DIGEST, Bd. 38, Nr. 2, 1. März 2006 (2006-03-01), Seiten 91-128, XP055101845, ISSN: 0583-1024, DOI: 10.1177/0583102406061499**
• **None**

**Beschreibung**

Die Erfindung betrifft ein Verfahren zur zerstörungsfreien und berührungslosen Ermittlung und Überwachung des Alterungs- und/oder Ermüdungszustands eines ein Elastomer enthaltenden Bauteils, bei dem mittels eines in das Elastomer eingebetteten Sensors und eines Sendeempfängers, welcher mit dem Sensor kommuniziert, die Permittivität des den Sender umgebenden Elastomers erfasst und ausgewertet wird. Die Erfindung betrifft außerdem eine Vorrichtung zur Durchführung dieses Verfahrens.

Bauteile mit funktionalen Komponenten aus Elastomeren werden in vielen Bereichen des Maschinen- und Fahrzeugbaus eingesetzt, beispielsweise in Luftfedern, Lagern, Transportbändern oder Zugmitteltrieben. Die darin verbauten elastomeren Werkstoffe leiden allerdings unter einer Verschlechterung ihrer mechanischen Eigenschaften durch Alterung, wenn sie längere Zeit äußeren Einflüssen ausgesetzt sind, sowie durch Materialermüdung, wenn sie im Betrieb statisch oder dynamisch belastet werden. Bauen die mechanischen Eigenschaften mit der Zeit ab, kann es zur Schädigung oder gar zum Versagen des Bauteils kommen. Dies ist mit einem wirtschaftlichen Schaden verbunden. Feste Austauschintervalle führen tendenziell zu unnötig hohen Servicekosten. Andererseits kann ein vorzeitiger Verschleiß möglicherweise unvermittelt zu einem Schaden führen. Wünschenswert ist es daher, die Alterung beziehungsweise Ermüdung des Elastomers rechtzeitig zu erkennen, um das Bauteil zu reparieren oder durch ein neues Bauteil zu ersetzen, so dass die Funktionalität und Sicherheit des Bauteils jederzeit gewährleistet ist. Für eine Analyse des Bauteils beziehungsweise Elastomers sind in der Regel weder ein Ausbau noch eine Materialentnahme praktikabel, um den Materialzustand zu bewerten. Daher werden Wege gesucht, die es ermöglichen, zerstörungsfrei und in situ Kennwerte für den Alterungs- und Ermüdungszustand dieser Bauteile zu ermitteln.

Eine Möglichkeit zur zerstörungsfreien Materialdiagnose von Elastomeren betrifft die dielektrischen Eigenschaften der Kautschuke und gummiähnlichen Werkstoffe. Solche dielektrischen Stoffe besitzen im Gegensatz zu den elektrisch leitenden Metallen keine freien Ladungsträger, so dass elektrische Felder auch im Inneren wirken. Phänomenologisch lässt sich dies beim Einbringen eines Dielektrikums zwischen die Platten eines Kondensators beobachten, wobei die Spannung des Kondensators sinkt und die Kapazität des Kondensators steigt. In einem von außen angelegten elektrischen Wechselfeld werden die elektrischen Ladungen im Dielektrikum, wie Elektronen, Ionen oder Dipole, auf mikroskopischer Ebene ausgelenkt. Es bildet sich ein Polarisationsfeld aus, welches dem äußern Wechselfeld entgegenwirkt. Die makroskopische Größe, welche den Polarisationszustand beschreibt und ein Maß für die Wechselwirkung des Materials mit dem elektrischen Feld darstellt, ist die Permittivität ε. Die Permittivität liefert Erkenntnisse über die Anzahl an Ladungen und deren Beweglichkeit im elektrischen Wechselfeld. Da vereinfacht dargestellt die Polarisierung der Ladungen stets hinter der Umpolung des anregenden Wechselfeldes zurückbleibt, also phasenverschoben ist, entstehen Verluste, die in einer komplexwertigen dielektrischen Funktion berücksichtigt werden. Die frequenzabhängige Antwort eines dielektrischen Materials, welches einem elektrischen Wechselfeld ausgesetzt ist, wird danach durch die komplexwertige dielektrische Funktion oder relative Permittivität $\varepsilon_r = \varepsilon_r' - i\varepsilon_r''$ beschrieben, wobei $\varepsilon_r = \dfrac{\varepsilon}{\varepsilon_0}$ die relative Permittivität des betrachteten dielektrischen Materials be-zogen auf die Elektrische Feldkonstante

$$\varepsilon_0 = \frac{10^7}{4\pi c^2} \approx 8{,}854 \cdot 10^{-12} \frac{As}{Vm}, \quad \varepsilon_r'$$ der Re-alteil

und $\varepsilon_r''$ der Imaginärteil, welcher die Verluste beschreibt, sind.

Im Falle eines isotropen Materials kann die Richtungsabhängigkeit der Permittivität als Tensor außer Betracht bleiben. Die Permittivität ist gegenüber einem anregenden Wechselfeld stark frequenzabhängig. Für die praktische Messbarkeit wird ein Sensor benötigt, über den mittels eines Sendeempfängers ein Wechselfeld in das zu untersuchende dielektrische Material eingespeist und eine Frequenzantwort ausgelesen sowie mittels eines Spektrometers dargestellt werden kann. Messergebnisse zeigen in der Regel Spektren, in denen der Realteil und/oder der Imaginärteil der relativen Permittivität über der Frequenz des anregenden elektrischen Feldes dargestellt sind. Es hat sich herausgestellt, dass die charakteristischen Spektren der dielektrischen Funktion eines dielektrischen Materials sich abhängig von dessen Alterungs- und Ermüdungszustands ändern.

Aus der Publikation "Hakim, I.K. et.al, Effekt of Natural Aging on the Dielectric Properties of Natural Rubber Mixed with Some White Fillers, Journal of Applied Polymer Science, 1977, Vol.21 (4), p. 1155-1158", ist eine Laboruntersuchung über den Einfluss der natürlichen Alterung von Prüfkörpern aus Naturkautschuk in Mischungen mit Siliziumdioxid oder Kalziumkarbonat aufgrund von Oxidationsvorgängen bei einer sechsjährigen Lagerung auf die dielektrische Funktion bekannt.

Die DE 103 05 110 B3 beschreibt ein Verfahren zur Feststellung wenigstens eines Zustandsparameters eines Dichtungselements, insbesondere einer Rohrverbindung, bei dem der Frequenzverlauf der dielektrischen Funktion eines dielektrischen Elements ausgewertet wird, um die Dichtheit, die korrekte Einbaulage und/oder den Alterungszustand der Dichtung zu überwachen. Das dielektrische Element besteht aus einer Mittelschicht aus dielektrischen Material sowie zwei Elektrodenschichten an beiden Seiten der Mittelschicht. Das dielektrische Element kann in das Dichtungselement eingebettet sein oder die Mittelschicht kann von dem Dichtungselement selbst gebildet sein, wobei die Elektroden an den Seitenflächen des Dichtungselements angeordnet sind. Die Elektrodenschichten sind mit einer Mess- und Auswerteeinrichtung elektrisch verbunden, welche elektrische Wechselfelder an die Elektrodenschichten anlegt, um in einem Frequenzbereich die dielektrische Funktion des dielektrischen Materials der Mittelschicht zu messen.

Aus der EP 2 375 099 A2 ist ein Elastomerbauteil, insbesondere eine Luftfeder mit einem Balg aus einem Elastomer bekannt, bei dem in dem Balg ein Sensor angeordnet ist, mittels dem die Dielektrizitätskonstante bzw. Permittivität des den Sensor umgebenden Elastomers messbar ist. Der Sensor besteht aus zwei spiralförmigen elektrischen Leiterbahnen, die ineinander gewunden und kreuzweise miteinander elektrisch verbunden sind. Die elektrischen Leiterbahnen sind mit einem Trägermaterial verbunden, welches in dem Balg eingebettet und mit diesem durch Vulkanisieren stoffschlüssig verbunden ist. Das Trägermaterial weist im Bereich des Sensors ähnliche mechanische Eigenschaften wie das umgebende Elastomer auf, wobei es jedoch mit Zusätzen versehen ist, welche die dielektrischen Eigenschaften des Elastomers so verändern, insbesondere dessen elektrische Leitfähigkeit erhöhen, so dass die Permittivität mit Hilfe des Sensors genau bestimmt werden kann. Der Sensor ist als ein LC-Schwingkreis aufgebaut, der als Alterungs- und Dehnungssensor bevorzugt in eine Rollfalte des Luftfederbalges eingebettet ist, wobei die Leiterbahnspiralen gleichzeitig die Spulen und den Kondensator bilden, zwischen denen das Dielektrikum liegt. Mittels eines Diagnosegerätes kann der Sensor, beispielsweise bei einem Wartungsservice, zu Schwingungen angeregt werden, wobei die Eigenfrequenz und die Dämpfungskonstante der erzwungenen Schwingung in dem Diagnosegerät analysiert und daraus der Realteil sowie der Imaginärteil der komplexwertigen relativen Dielektrizitätsfunktion bestimmt werden. Aus dem Realteil und dem Imaginärteil kann durch Vergleich mit einer Referenzkennlinie, welche an einer neuwertigen Luftfeder oder einem anderen Prüfkörper aufgenommen wurde, auf den Alterungszustand des Elastomers geschlossen werden.

Die US 3 906 340 A offenbart einen in einem zu prüfenden Material angeordneten Schwingkreis, bestehend aus einer Induktivität (Spule) und einer Kapazität (Kondensator), bei der die Spule bildende Leiter an das zu prüfende Material angepasst werden kann.

Die DE 10 2010 016359 A1 offenbart einen Schwingkreis aus einer Induktivität und einer Kapazität, wobei jedoch die Spule direkt im Gummi eingelassen ist und gleichzeitig als Kapazität dient.

Der Erfindung liegt die Aufgabe zugrunde, ein Verfahren zur zerstörungsfreien und berührungslosen Ermittlung sowie Überwachung des Alterungs- und/oder Ermüdungszustands eines ein Elastomer enthaltenden Bauteils vorzustellen, welches einfach und zuverlässig ist. Ferner liegt der Erfindung die Aufgabe zugrunde eine Vorrichtung vorzustellen, die eine genaue, einfache, zuverlässige, zerstörungsfreie und berührungslose Ermittlung sowie Überwachung des Materialzustands eines Elastomerbauteils anhand seiner dielektrischen Eigenschaften ermöglicht.

Die Lösung dieser Aufgaben ergibt sich aus den Merkmalen der unabhängigen Ansprüche, während vorteilhafte Ausgestaltungen und Weiterbildungen der Erfindung den Unteransprüchen entnehmbar sind.

Dielektrische Eigenschaften können als Materialkennwerte für Änderungen in der makromolekularen Struktur eines Festkörpers dienen. Im Gegensatz zum elektrischen Widerstand beziehungsweise zur elektrischen Leitfähigkeit von Stoffen lässt sich bei Dielektrika die komplexwertige dielektrische Funktion mittels elektrischer Wechselfelder berührungslos messen.

Der Erfindung liegt die Erkenntnis zugrunde, dass sich bei einem Elastomerprodukt der Materialzustand einer Gummimischung, welche unter dem Einfluss von Ermüdung und/oder Alterung steht, anhand der dielektrischen Eigenschaften des Materials ermitteln und überwachen lässt. Messergebnisse an Prüflingen haben gezeigt, dass sich die relativen Permittivität in einem weiten Frequenzbereich anregender elektrischer Wechselfelder unterhalb des MHz-Bereiches als alterungsempfindlicher und ermüdungsempfindlicher Kennwert von Gummimischungen eignet. In einem betrachteten Frequenzbereich sind die Frequenzverläufe der relativen Permittivität je nach Ermüdungszustand des Elastomers signifikant unterschiedlich. Die Ermittlung und Überwachung des Materialzustands kann mittels einer resonanten Struktur, die in das Elastomerprodukt integriert ist sowie einem Sendeempfänger zerstörungsfrei und berührungslos erfolgen. Die resonante Struktur kann als eine Sensorantenne wirken, mittels der gemessen werden kann, wie eine aktuelle Frequenz von einer Resonanzfrequenz der resonanten Struktur in Folge von Materialalterung und/oder Materialermüdung abweicht. Die Antenne selbst kann, leitfähig modifiziert, jedoch mit nahezu denselben mechanischen Eigenschaften, aus dem gleichen Elastomer bestehen, wie dasjenige Material, in das die Antenne eingebettet ist.

Die Erfindung geht daher aus von einem Verfahren zur zerstörungsfreien und berührungslosen Ermittlung sowie Überwachung des Alterungs- und/oder Ermüdungszustands eines ein Elastomer enthaltenden Bauteils, bei dem mittels eines in das Elastomer eingebetteten Sensors und eines Sendeempfängers, welcher mit dem Sensor kommuniziert, die Permittivität des den Sensor umgebenden Elastomers erfasst und ausgewertet wird. Zur Lösung der gestellten Aufgabe hinsichtlich des Verfahrens sieht die Erfindung vor, dass in einem neuwertigen Zustand des Bauteils die dielektrischen Materialeigenschaften des eingebetteten Sensors und des den Sensor umgebenden Elastomers resonant aufeinander abgestimmt sind, so dass eine von dem Sendeempfänger ausgesandte elektromagnetische Prüfwelle festgelegter Anregungsfrequenz mit einer Resonanz des abgestimmten Sensors beantwortet wird, und dass in einem gealterten und/oder ermüdeten Zustand des Bauteils anhand einer Frequenzverstimmung der Antwort des Sensors gegenüber der Prüfwelle auf den gealterten und/oder ermüdeten Materialzustand des den Sensor umgebenden Elastomers sowie daraus auf den Alterungs- und Ermüdungszustand des Bauteils geschlossen wird.

Hinsichtlich der Lösung der vorrichtungsbezogenen Aufgabe geht die Erfindung aus von einer Vorrichtung zur zerstörungsfreien und berührungslosen Ermittlung sowie Überwachung des Alterungs- und/oder Ermüdungszustands eines ein Elastomer enthaltenden Bauteils, bei der mittels eines in das Elastomer eingebetteten Sensors und eines Sendeempfängers, welcher mit dem Sensor kommuniziert, die Permittivität des den Sensor umgebenden Elastomers erfassbar und auswertbar ist. Bei dieser Vorrichtung ist zur Lösung der Aufgabe vorgesehen, dass der Sensor eine resonante Struktur oder einen Resonator aufweist, bei dem die Resonanzfrequenz von der Permittivität des den Sensor umgebenden Elastomers des zu überwachenden Bauteils abhängig ist, und dass Mittel für eine Resonanzfrequenzanalyse der zu empfangenden Signale des Sensors vorhanden sind, welche so ausgebildet sind, dass mittels einer Resonanzfrequenzanalyse der Alterungs- und/oder Ermüdungszustand des Elastomers des Bauteils indirekt feststellbar ist.

Unter einem Elastomer wird ein synthetischer oder natürlicher Kautschuk oder gummiähnlicher Werkstoff mit dielektrischen Materialeigenschaften verstanden.

Demnach schlägt die Erfindung vor, die Änderung der dielektrischen Eigenschaft des zu überwachenden Bauteils auf Grund von Alterung und/oder Ermüdung zur Verstimmung einer Resonanzfrequenz zu verwenden, die sich mittels eines Sensors detektieren, an einen Sendeempfänger übertragen sowie mittels einer Resonanzfrequenzanalysevorrichtung auswerten lässt.

Dies wird dadurch ermöglicht, dass eine resonante Struktur oder ein Resonator in das zu überwachende Elastomerbauteil eingebettet wird, so dass die Resonanzfrequenz von der Permittivität des den Sensor umgebenden Elastomers des zu überwachenden Bauteils abhängt. Eine solche resonante Struktur besitzt eine relativ scharfe Resonanzfrequenz. Die Resonanzfrequenz ist sehr empfindlich gegenüber Änderungen der dielektrischen Eigenschaften. Die Messung einer Frequenzverstimmung ist somit ein empfindlicher Indikator für Änderungen der dielektrischen Eigenschaften, wie sie aufgrund von Materialalterung und/oder Materialermüdung auftreten.

Grundsätzlich kann die Erfindung bei allen Anwendungen, in denen die Verstimmung einer resonanten Struktur durch Änderung der dielektrischen Eigenschaften des einbettenden elastomeren Materials gewünscht wird, verwendet werden. Beispielsweise kann vorgesehen sein, dass sie bei einem Luftfedersystem eines Fahrzeugs mit Bälgen, welche aus einer Gummimischung bestehen, angewandt wird, wobei der Materialzustand der Gummimischung der Bälge unter dem Einfluss der Materialermüdung durch mechanisch-dynamische und thermische Beanspruchung sowie unter dem Einfluss der Materialalterung regelmäßig und/oder bedarfsweise zerstörungsfrei und berührungslos anhand der dielektrischen Eigenschaften des Materials im Produkt mittels einer resonanten Struktur und eines Sendeempfängers überprüft wird.

Zusätzlich zur Ermüdung und Alterung des Elastomerbauteils in Folge dynamisch-mechanischer Beanspruchung lassen sich auch andere Ursachen, welche die Polarisationsmechanismen und damit die dielektrischen Eigenschaften des Elastomers beziehungsweise der Gummimischung beeinflussen, überwachen, beispielsweise ein thermischer und/oder oxidativer Abbau der Polymerstruktur oder bestimmte Mischungsbestandteile der Gummimischung.

Das Sensorkonzept sieht vor, dass eine resonante Struktur in das Produkt eingebettet wird. Gemäß der Erfindung kann vorgesehen sein, dass der Sensor aus dem gleichen Elastomer oder aus einem ähnlichen Elastomer mit vergleichbaren mechanischen Eigenschaften wie das ihn umgebende Elastomer des zu überwachenden Bauteils besteht, wobei das Elastomer des Sensors dielektrisch, insbesondere elektrisch leitfähig, modifiziert ist. Dadurch kann die dielektrische Eigenschaftsänderung des zu überwachenden Bauteils aufgrund von Alterung und/oder Ermüdung direkt überwacht werden, wodurch jederzeit sehr genaue und zuverlässige Aussagen über den tatsächlichen Zustand des Bauteils möglich sind. An dieser Stelle sei erwähnt, dass grundsätzlich auch eine herkömmliche Antenne aus Metall verwendet werden kann.

Der Sensor der erfindungsgemäßen Vorrichtung ist als eine Dipolantenne ausgebildet, die aus einem Elastomer besteht und deren Resonatorlänge

$$l = \frac{\lambda_{res}}{2\sqrt{\varepsilon_h}} \cdot m$$

(m =1,2,...) ist, mit der Resonanzwellenlänge $\lambda_{res}$ und der Permitti-vität $\varepsilon_h$ des die Dipolantenne umgebenden Elastomers des zu überwachenden Bauteils. Die Resonanzwellenlänge ist gemäß der Gleichung

$$\lambda_{res} = \frac{c}{f_{res}}$$

über die Ausbreitungsgeschwindigkeit c der elektromagnetischen Welle mit dem Kehrwert der Resonanzfrequenz $f_{res}$ verbunden.

Die Dipolantenne kann demnach aus dem Elastomer bestehen, in das sie eingebettet ist, oder aus einem ähnlichen Elastomer bestehen, wobei das Antenennmaterial leitfähig modifiziert ist. Die resonante Struktur besteht beispielsweise aus einem stark leitfähig modifizierten Gummi, dessen Grundmischung zum Beispiel dem genutzten Kautschuktyp des umgebenden Gummis in seinen mechanischen Eigenschaften ähnelt. Die Einbettung der Dipolantenne in das umgebende Gummi kann durch Vulkanisation erfolgen.

Die Dipolantenne kann als eine passive oder als eine aktiv bestromte Antenne ausgebildet sein.

Das Elastomer der Antenne besitzt durch die Modifizierung einen geringeren spezifischen Gleichspannungswiderstand gegenüber dem Elastomer, dessen Alterung anhand der dielektrischen Permittivität überwacht werden soll. Durch die dielektrische Modifizierung des Antennenmaterials wird eine hohe Empfindlichkeit bei der Messung der Permittivität mittels eines Sendeempfängers, insbesondere im Falle einer aktiv bestromten Dipolantenne, erreicht.

In der neuwertigen beziehungsweise ungealterten Konfiguration sind die Materialeigenschaften der eingebetteten resonanten Struktur der Dipolantenne und die des umgebenden Elastomers gemäß der Erfindung resonant zueinander abgestimmt, so dass eine einfallende elektromagnetische Prüfwelle festgelegter Anregungsfrequenz beziehungsweise festgelegter Anregungswellenlänge der Resonanzfrequenz beziehungsweise der Resonanzwellenlänge der Dipolantenne entspricht. Im Ergebnis wird in der ungealterten Konfiguration eine starke Wechselwirkung des Elastomers des Bauteils beziehungsweise der Antenne mit der Prüfwelle durch einen Resonanzpeak der Amplitude im Frequenzspektrum der Antenne beobachtet. Der Resonanzpeak kann im Frequenzspektrum des Imaginärabteils der relativen Permittivität überwacht werden. Es ist auch möglich, die spektrale Position einer Verlustbande im Frequenzverlauf des Realteils der relativen Permittivität zu überwachen. Hierbei ist diese spektrale Position als Stufe im Frequenzverlauf erkennbar.

In Untersuchungen hat sich herausgestellt, dass eine Alterung und/oder Ermüdung des Materials die dielektrischen Eigenschaften derart verändert, dass die Permittivität des die Dipolantenne umgebenden Elastomers des zu überwachenden Bauteils kleiner wird und die effektive Resonatorlänge der resonanten Struktur gemäß der obigen Beziehung somit zunehmen müsste. Dadurch ist die resonante Struktur verstimmt und die Wechselwirkung mit dem anregenden elektromagnetischen Feld wird geschwächt oder ist ganz verschwunden.

Durch eine Alterung und/oder Ermüdung des Bauteils können sich nicht nur die dielektrischen Eigenschaften des die Dipolantenne umgebenden Elastomers des zu überwachenden Bauteils sondern auch die dielektrischen Eigenschaften der resonanten Struktur der Dipolantenne selbst ändern, wodurch sich ein zusätzlicher Beitrag zur Verstimmung der Resonanzfrequenz ergibt.

Die Prüfwelle lässt sich mittels eines Sendeempfängers aus einer Entfernung erzeugen und nach der Reflexion durch die Materialantwort verändert wieder detektieren, um den Materialzustand abzufragen. Die Abfrage des Materialzustands eines zu überwachenden Bauteils eines Fahrzeugs kann somit beispielsweise während eines ohnehin vorgesehenen Service-Vorgangs erfolgen. Ein Ausbau des zu prüfenden Bauteils ist dazu nicht erforderlich.

Eine Möglichkeit dazu bietet die Transponder-Technologie. Beispielsweise kann vorgesehen sein, dass ein Transponder angeordnet ist, der mit dem Sensor wirkverbunden ist, und dass der Sendeempfänger ein Transponder-Lesegerät ist. Demnach kann eine sogenannte RFID (radio-frequency identifi-cation)-Kennzeichnung verwendet werden, welche aus einem Etikett als Transponder, der an der Dipolantenne angeordnet ist und einem entfernten Transponder-Lesegerät besteht. Die RFID-Kennzeichnung erzeugt selbst kein Feld, sondern beeinflusst über die resonante Struktur der Antenne aus Gummi das elektromagnetische Sendefeld des Lesegerätes. Je nach dielektrischem Zustand des Antennenmaterials wird das Sendefeld unterschiedlich stark beeinflusst. Eine mit der Transponder-Technologie funktionierende Antenne auf Basis einer leitfähig modifizierten Gummimischung konnte bereits bei einer Frequenz im Megahertzbereich realisiert werden.

Gemäß weiterer Ausführungsformen der Vorrichtung kann vorgesehen sein, dass der Sensor als eine Mikrostreifenantenne (stripline) oder als eine Patchantenne ausgebildet ist. Eine Patchantenne ist über Mikrostreifenzuleitungen mit einer Betriebsspannung versorgbar. Bei beiden Antennenvarianten ist vorgesehen, dass das den Sensor umgebende Elastomer des zu überwachenden Bauteils als ein Substrat des Sensors fungiert. Dabei kann das Substrat auf einer dünnen Grundplatte angeordnet sein.

Patchantennen sind besonders gut auf leiterplattenartigen Substraten integrierbar und können ähnlich wie Dipolantennen als resonante Struktur wirken. Mikrostreifenantennen können als dünne leitfähige Streifen auf einem Dielektrikum aufgebracht werden und wirken als elektrische Wellenleiter. Sie sind relativ kostengünstig herstellbar. Eine solche Patchantenne oder Mikrostreifenantenne kann als eine Sendeantenne bestromt und angesteuert werden, wobei die Antennenabstrahlung meist eine Vorzugsrichtung hat. Eine durch Alterung und/oder Ermüdung abnehmende relative Permittivität resultiert bei solchen Antennen darin, dass die Reichweite der abgestrahlten elektromagnetischen Welle zunimmt und eine bei festem Abstand des Empfängers detektierte Signalstärke ein Maß für den Ermüdungszustand des zu überwachenden Elastomerbauteils darstellt. Patchantennen und Mikrostreifenantennen stellen somit bei der Verstimmung der Resonanzfrequenz eine entfernungsabhängige Messgröße für den Materialzustand des zu überwachenden Bauteils zur Verfügung.

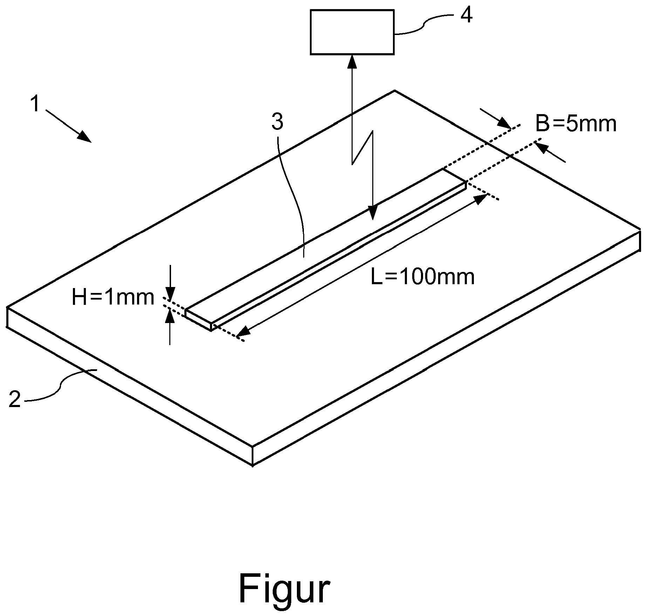

Die Erfindung wird nachfolgend anhand der beiliegenden Zeichnung an einer Ausführungsform weiter erläutert. Darin zeigt die einzige Figur eine stark schematisierte Vorrichtung zur zerstörungsfreien und berührungslosen Ermittlung sowie Überwachung des Alterungs- und/oder Ermüdungszustands eines ein Elastomer enthaltenden Bauteils.

Demnach weist das Bauteil 1 ein Elastomer auf, welches als Träger 2 für einen Sensor 3 zur Ermittlung und Überwachung des Alterungs- und/oder Ermüdungszustands des Bauteils 1 fungiert. Bei dem Bauteil 1 kann es sich beispielsweise um eine Luftfeder eines Fahrzeugs handeln, mit einem Balg, bestehend aus einem dielektrischen Elastomer, insbesondere einer Gummimischung, wie sie für solche Bälge üblicherweise verwendet wird. Der Sensor 3 kann in einer Rollfalte des Balgs eingebettet sein. In der Figur ist der Balg beziehungsweise die Rollfalte durch den Träger 2 angedeutet.

Der Sensor 3 ist als eine streifenförmige Dipolantenne ausgebildet. Lediglich zur Verdeutlichung der Größenordnung sind beispielhaft die Maße Länge L = 100 mm, Breite B = 5 mm und Höhe beziehungsweise Dicke H = 1 mm der Dipolantenne 3 angegeben.

Die Dipolantenne 3 weist eine resonante Struktur beziehungsweise einen Resonator auf, der aus der gleichen Gummimischung wie der Träger 2 besteht. Das Antennenmaterial ist allerdings stark elektrisch leitfähig modifiziert, beispielsweise durch Einbringung von Zusätzen, wie elektrisch leitfähigen Partikeln. Das Material der Dipolantenne 3 besitzt dementsprechend einen relativ geringen spezifischen Gleichspannungswiderstand $\rho_a^{DC}$. Das Material des Trägers 2 besitzt einen vergleichsweise hohen spezifischen Gleichspannungswiderstand $\rho_h^{DC}$. Die Dipolantenne 3 ist vorzugsweise durch Vulkanisation in das Material des umgebenden Trägers 2 eingebettet und von außen, beispielsweise induktiv, elektrisch bestrombar.

Eine Alterung und/oder Ermüdung des Bauteils 1 soll anhand der Änderung der dielektrischen Eigenschaften des Trägers 2 aufgrund von mechanischdynamischer Belastung oder Alterung, hervorgerufen durch die betriebsüblichen Lastwechsel, welche im Betrieb des Fahrzeugs ständig auftreten, sowie durch Umwelteinflüsse, ermittelt werden.

Dazu wird mittels eines Sendeempfängers 4, beispielsweise ein Transponder-Lesegerät, welches mit einem nicht dargestellten Transponder an der Dipolantenne 3 korrespondiert, ein Wechselfeld in das Bauteil 1 eingestrahlt, welches als eine Prüfwelle zur Abfrage des Materialzustands des Trägers 2 fungiert.

In der ungealterten Konfiguration sind die Materialeigenschaften der eingebetteten Struktur, also der Dipolantenne 3 und des umgebenden Elastomers, also des Trägers 2, resonant aufeinander abgestimmt, so dass die einfallende elektromagnetische Prüfwelle festgelegter Anregungsfrequenz und Anregungswellenlänge der Resonanzfrequenz und Resonanzwellenlänge der abgestimmten Dipolantenne 3 entspricht. Die Resonatorlänge der Dipolantenne 3 ist dann:

$$l = \frac{\lambda_{res}}{2\sqrt{\varepsilon_h}} \cdot m \, ,$$

(m =1,2,...), mit der Resonanzwellenlänge $\lambda_{res}$ und der Permittivität $\varepsilon_h$ des die Dipolantenne umgebenden Elastomers des zu untersuchenden Trägers 2. Die Resonatorlänge 1 entspricht der Länge L der Dipolantenne 3.

Durch die resonante Abstimmung erfolgt eine starke Wechselwirkung zwischen dem einstrahlenden Wechselfeld und der resonanten Struktur, die durch den Sendeempfänger 4 detektiert wird. Eine Ermüdung und/oder Alterung des Materials des Trägers 2 (und des Materials der Antenne 3 selbst), verändert die dielektrischen Eigenschaften so, dass die relative Permittivität

$$\varepsilon_h = \varepsilon_h' - i\varepsilon_h''$$

des Trägers 2 abnimmt, wodurch die Resonatorlänge 1 eigentlich zunehmen müsste, um die resonante Abstimmung mit der festgelegten Prüfwelle zu erhalten. Da die Resonatorlänge 1 gleich bleibt, ist die resonante Struktur verstimmt. In der Folge ist die Wechselwirkung mit dem anregenden elektromagnetischen Prüffeld geschwächt oder ganz verschwunden, was wiederum von dem Sendeempfänger 4 detektiert beziehungsweise registriert wird. Die Auswertung der empfangenen Signale kann mittels eines tragbaren Spektralanalysators (beispielsweise FieldFox RF Analyzer der Fa. Agilent Technologies) erfolgen. Damit kann die spektrale Position der Resonanz festgestellt und diese einem Alterungs- beziehungsweise Ermüdungszustand des Elastomers zugeordnet werden.

Mittels der Dipolantenne 3 und dem Sendeempfänger 4 kann somit eine Prüfwelle erzeugt und nach einer Reflexion durch das zu untersuchende Elastomer 2 verändert detektiert werden, um den Materialzustand berührungslos und zerstörungsfrei abzufragen. Dadurch kann nach einer entsprechenden Auswertung auf den aktuellen Alterungs- und/oder Ermüdungszustands des elastomerhaltigen Bauteils 1 geschlossen werden.

**Bezugszeichenliste**

| | |
|---|---|
| 1 | Bauteil |
| 2 | Träger, Elastomer |
| 3 | Sensor, Dipolantenne |
| 4 | Sendeempfänger, Transponder-Lesegerät |
| B | Breite |
| H | Höhe, Dicke |
| L | Länge |
| l | Resonatorlänge |
| ε | Permittivität |
| ε' | Permittivität Realteil |
| c | Ausbreitungsgeschwindigkeit |
| f | Frequenz |
| $f_{res}$ | Resonanzfrequenz |

λ     Wellenlänge
$\rho^{DC}$   spezifischer Gleichspannungswiderstand
a     Antenne
r     relativ
res   Resonanz

**Patentansprüche**

1. Verfahren zur zerstörungsfreien und berührungslosen Ermittlung und Überwachung des Alterungs- und/oder Ermüdungszustands eines ein Elastomer (2) in Form einer Gummimischung enthaltenden und als Luftfederbalges in einem Luftfedersystem eines Fahrzeugs ausgebildeten Bauteils (1), bei dem mittels eines in das Elastomer (2) eingebetteten Sensors (3) und eines Sendeempfängers (4), welcher mit dem Sensor (3) kommuniziert, die Permittivität des den Sensor (3) umgebenden Elastomers (2) erfasst und ausgewertet wird, wobei der eingebettete Sensor leitfähig modifiziert ist und aus dem gleichen Elastomer oder aus einem ähnlichen Elastomer besteht und dass in einem neuwertigen Zustand des Bauteils (1) die dielektrischen Materialeigenschaften des eingebetteten Sensors (3) und des den Sensor (3) umgebenden Elastomers (2) resonant aufeinander abgestimmt sind, so dass eine von dem Sendeempfänger (4) ausgesandte elektromagnetische Prüfwelle festgelegter Anregungsfrequenz mit einer Resonanz des abgestimmten Sensors (3) beantwortet wird, und dass in einem gealterten und/oder ermüdeten Zustand des Bauteils (1) anhand einer Frequenzverstimmung der Antwort des Sensors (3) gegenüber der Prüfwelle auf den gealterten und/oder ermüdeten Materialzustand des den Sensor (3) umgebenden Elastomers (2) und daraus auf den Alterungs- und Ermüdungszustand des Bauteils (1) geschlossen wird und der Materialzustand der Gummimischung der Bälge unter dem Einfluss der Materialermüdung durch mechanisch-dynamische und thermische Beanspruchung sowie unter dem Einfluss der Materialalterung regelmäßig und/oder bedarfsweise überprüft wird.

2. Vorrichtung zur zerstörungsfreien und berührungslosen Ermittlung sowie Überwachung des Alterungs- und/oder Ermüdungszustands eines ein Elastomer (2) enthaltenden Bauteils (1), bei der mittels eines in das Elastomer (2) eingebetteten leitfähig modifizierten Sensors (3) und eines Sendeempfängers (4), welcher mit dem Sensor (3) kommuniziert, die Permittivität des den Sensor (3) umgebenden Elastomers (2) erfassbar und auswertbar ist, wobei der Sensor (3) eine resonante Struktur oder einen Resonator aufweist, bei dem die Resonanzfrequenz von der Permittivität des den Sensor (3) umgebenden Elastomers (2) des zu überwachenden Bauteils (1) abhängig ist, und dass Mittel für eine Resonanzfrequenzanalyse der zu empfangenden Signale des Sensors vorhanden sind, welche so ausgebildet sind, dass mittels einer Resonanzfrequenzanalyse der Alterungs- und/oder Ermüdungszustand des Elastomers des Bauteils indirekt feststellbar ist, wobei der eingebettete Sensor (3) aus dem gleichen Elastomer oder aus einem ähnlichen Elastomer mit vergleichbaren mechanischen Eigenschaften wie das ihn umgebende Elastomer (2) des zu überwachenden Bauteils (1) besteht und in einem neuwertigen Zustand des Bauteils (1) die dielektrischen Materialeigenschaften des eingebetteten Sensors (3) und des den Sensor (3) umgebenden Elastomers (2) resonant aufeinander abgestimmt sind, wobei das Elastomer des Sensors (3) dielektrisch dadurch modifiziert ist, dass die Leitfähigkeit des Sensormaterials im Vergleich zum Material des Bauteils verändert ist, und wobei der eingebettete Sensor (3) als eine Dipolantenne ausgebildet ist, die aus einem Elastomer besteht und deren Resonatorlänge sich

$$l = \frac{\lambda_{res}}{2\sqrt{\varepsilon_h}} \cdot m$$

aus der Gleichung (m = 1,2,...) ergibt, mit der Resonanzwellenlänge $\lambda_{res}$ und der Permittivität $\varepsilon_h$ des die Dipolantenne umgebenden Elastomers (2) des zu überwachenden Bauteils (1).

3. Vorrichtung nach Anspruch 2, **dadurch gekennzeichnet, dass** ein Transponder angeordnet ist, der mit dem Sensor (3) wirkverbunden ist, und dass der Sendeempfänger (4) ein Transponder-Lesegerät ist.

4. Vorrichtung nach einem der Ansprüche 2 oder 3, **dadurch gekennzeichnet, dass** der Sensor (3) als eine Mikrostreifenantenne oder als eine Patchantenne ausgebildet ist, wobei die Patchantenne über Mikrostreifenzuleitungen mit einer Betriebsspannung versorgbar ist, und dass bei beiden Antennenvarianten vorgesehen ist, dass das den Sensor (3) umgebende Elastomer des zu überwachenden Bauteils als ein Substrat des Sensors fungiert.

**Claims**

1. Method for the non-destructive and contactless determination and monitoring of the state of aging and/or fatigue of a component (1) containing an elastomer (2) in the form of a rubber compound and formed as an air spring bellows in an air spring system of a vehicle, in which method, by means of a sensor (3) embedded in the elastomer (2) and a transceiver (4), which communicates with the sensor (3), the permittivity of the elastomer (2) surrounding the sensor (3) is detected and evaluated, wherein the embedded sensor is conductively modified and consists of the same elastomer or a similar elastomer and wherein, in an as-new state of the component (1), the dielectric material properties of the embedded sensor (3) and of the elastomer (2) surrounding the sensor (3) are resonantly tuned to one another, so that an electromagnetic test wave of a fixed excitation frequency emitted by the transceiver (4) is responded to by a resonance of the tuned sensor (3), and wherein, in an aged and/or fatigued state of the component (1), the aged and/or fatigued state of the material of the elastomer (2) surrounding the sensor (3) is deduced on the basis of frequency mistuning of the response of the sensor (3) with respect to the test wave, and from it the state of aging and fatigue of the component (1) is deduced, and the state of the material of the rubber compound of the bellows under the influence of material fatigue due to mechanical-dynamic and thermal stressing and under the influence of the aging of the material is checked regularly and/or whenever required.

2. Device for the non-destructive and contactless determination and monitoring of the state of aging and/or fatigue of a component (1) containing an elastomer (2), in which device, by means of a conductively modified sensor (3) embedded in the elastomer (2) and a transceiver (4), which communicates with the sensor (3), the permittivity of the elastomer (2) surrounding the sensor (3) can be detected and can be evaluated, wherein the sensor (3) has a resonant structure or a resonator, in which the resonant frequency is dependent on the permittivity of the elastomer (2) of the component (1) to be monitored that is surrounding the sensor (3), and wherein means for a resonant frequency analysis of the signals of the sensor to be received are present and are formed in such a way that the state of aging and/or fatigue of the elastomer of the component can be indirectly established by means of a resonant frequency analysis, wherein the embedded sensor (3) consists of the same elastomer or of a similar elastomer with comparable mechanical properties as the elastomer (2) of the component (1) to be monitored that is surrounding it and, in an as-new state of the component (1), the dielectric material properties of the embedded sensor (3) and of the elastomer (2) surrounding the sensor (3) are resonantly tuned to one another, wherein the elastomer of the sensor (3) is dielectrically modified by the conductivity of the sensor material being changed in comparison with the material of the component, and wherein the embedded sensor (3) is formed as a dipole antenna which consists of an elastomer and the resonator length of which is obtained from the equation

$$ l = \frac{\lambda_{res}}{2\sqrt{\varepsilon_h}} \cdot m $$

(m=1,2,...) with the resonant wavelength $\lambda_{res}$ and the permittivity $\varepsilon_h$ of the elastomer (2) of the component (1) to be monitored that is surrounding the dipole antenna.

3. Device according to Claim 2, **characterized in that** a transponder that is operatively connected to the sensor (3) is arranged, and **in that** the transceiver (4) is a transponder reader.

4. Device according to either of Claims 2 and 3, **characterized in that** the sensor (3) is formed as a microstrip antenna or as a patch antenna, wherein the patch antenna can be supplied with an operating voltage by way of microstrip leads, and **in that** in both variants of the antenna it is provided that the elastomer of the component to be monitored that is surrounding the sensor (3) acts as a substrate of the sensor.

**Revendications**

1. Procédé de détermination et de surveillance non destructives et sans contact de l'état de vieillissement et/ou de fatigue d'un composant (1) qui contient un élastomère (2) se présentant sous la forme d'un mélange de caoutchoucs et qui est réalisé sous la forme d'un soufflet de suspension pneumatique dans un système de suspension pneumatique d'un véhicule, procédé dans lequel la permittivité de l'élastomère (2) entourant le capteur (3) est détectée et évaluée à l'aide d'un capteur (3) incorporé à l'élastomère (2) et d'un émetteur-récepteur (4) qui communique avec le capteur (3), le capteur incorporé étant modifié en termes de conduction et comprenant le même élastomère ou un élastomère similaire et les propriétés diélectriques du matériau du capteur incorporé (3) et de l'élastomère (2) entourant le capteur (3) étant adaptées les unes aux autre de manière résonante lorsque le composant (1) est comme neuf de sorte qu'une onde électromagnétique de test de fréquence d'excitation spécifiée, émise par l'émetteur-récepteur (4), reçoive en réponse une résonance du capteur (3) adapté, et une conclusion quant à l'état du matériau vieilli et/ou fatigué de l'élastomère (2) entourant le capteur (3) et à partir de là quant à l'état de vieillissement et de fatigue du composant (1) étant tirée sur la base d'un désaccord en fréquence de la réponse du capteur (3) par rapport à l'onde de test lorsque le composant (1) est vieilli et/ou fatigué et l'état du matériau du mélange de caoutchoucs du soufflet étant vérifié régulièrement et/ou selon les besoins sous l'influence de la fatigue du matériau par le biais de contraintes mécano-dynamiques et thermiques et sous l'influence du vieillissement du matériau.

2. Dispositif de détermination et de surveillance non destructives et sans contact de l'état de vieillissement et/ou de fatigue d'un composant (1) contenant un élastomère (2), procédé dans lequel la permittivité de l'élastomère (2) entourant le capteur (3) pouvant être détectée et évaluée à l'aide d'un capteur (3) modifié en termes de conduction et incorporé dans l'élastomère (2) et d'un émetteur-récepteur (4) qui communique avec le capteur (3), le capteur (3) comportant une structure résonante ou un résonateur dans lequel la fréquence de résonance dépend de la permittivité de l'élastomère (2), entourant le capteur (3), du composant (1) à surveiller, et des moyens étant prévus pour effectuer une analyse de fréquence de résonance des signaux à recevoir du capteur, lesquels moyens sont conçus de telle sorte que l'état de vieillissement et/ou de fatigue de l'élastomère du composant puisse être déterminé indirectement à l'aide d'une analyse de fréquence de résonance, le capteur incorporé (3) comprenant le même élastomère ou un élastomère similaire ayant des propriétés mécaniques comparables à celles de l'élastomère environnant (2) du composant à surveiller et, lorsque le composant (1) est comme neuf, les propriétés diélectriques du matériau du capteur incorporé (3) et de l'élastomère (2) entourant le capteur (3) étant adaptées les unes aux autres de manière résonante, l'élastomère du capteur (3) étant modifié du point de vue diélectrique du fait que la conductivité du matériau du capteur est modifiée par rapport au matériau du composant (1), et le capteur incorporé (3) étant réalisé sous la forme d'une antenne dipôle qui comprend un élastomère et dont la longueur de résonateur résulte de l'équation

$$l = \frac{\lambda_{res}}{2\sqrt{\varepsilon_h}} \cdot m$$

(m = 1,2,...), $\lambda_{res}$ étant la longueur d'onde de résonance et $\varepsilon_h$ étant la permittivité de l'élastomère (2), entourant l'antenne dipôle, du composant à surveiller (1).

3. Dispositif selon la revendication 2, **caractérisé en ce qu'**un transpondeur est prévu qui est relié fonctionnellement au capteur (3), et **en ce que** l'émetteur-récepteur (4) est un lecteur de transpondeur.

4. Dispositif selon l'une des revendications 2 ou 3, **caractérisé en ce que** le capteur (3) est réalisé sous la forme d'une antenne micro-ruban ou d'une antenne patch, l'antenne patch pouvant être alimentée en tension de fonctionnement via des conducteurs micro-rubans, et **en ce que**, pour les deux variantes d'antenne, il est prévu que l'élastomère, entourant le capteur (3), du composant à surveiller fonctionne comme un substrat du capteur.

Figur

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10305110 B3
- EP 2375099 A2
- US 3906340 A
- DE 102010016359 A1


**In der Beschreibung aufgeführte Nicht-Patentliteratur**

- **HAKIM, I.K.** Effekt of Natural Aging on the Dielectric Properties of Natural Rubber Mixed with Some White Fillers. *Journal of Applied Polymer Science,* 1977, vol. 21 (4), 1155-1158